# EUROPEAN PATENT SPECIFICATION

(11) **EP 2 249 736 B1**
(45) Date of publication and mention of the grant of the patent: **15.08.2018**
(21) Application number: 09726364.4
(22) Date of filing: 27.03.2009
(51) Int. Cl.: A61B 34/00, A61B 34/30

(54) **MODEL CATHETER INPUT DEVICE**
MODELLKATHETEREINFÜHRUNGSVORRICHTUNG
DISPOSITIF D'ENTRÉE DE CATHÉTER MODÉLISÉ

(30) Priority: 27.03.2008 US 40141 P; 27.03.2008 US 40142 P; 27.03.2008 US 40143 P; 24.09.2008 US 99904 P; 31.12.2008 US 347442
(43) Date of publication of application: 17.11.2010
(73) Proprietor: St. Jude Medical, Atrial Fibrillation Division, Inc., St. Paul, Minnesota 55117-9913 (US)
(72) Inventor: KIRSCHENMAN, Mark B., Waverly MN 55390 (US)
(74) Representative: Kramer Barske Schmidtchen Patentanwälte PartG mbB
(86) International application number: PCT/US2009/038536
(87) International publication number: WO 2009/120948

(56) References cited:
- WO-A1-2006/120666
- WO-A1-2008/136160
- GB-A- 2 397 177
- US-A- 3 605 725
- US-A- 5 318 525
- US-A- 5 545 200
- US-A- 5 800 178
- US-A- 6 096 004
- US-A1- 2002 068 868
- US-A1- 2004 193 239
- US-A1- 2005 016 316
- US-A1- 2007 043 338
- US-A1- 2008 009 791
- US-B1- 6 348 911

## Description

### BACKGROUND

### a. Field

The present disclosure relates to robotic catheter systems, and more particularly, to improved devices for controlling movement of robotic catheter systems and sheaths within a treatment area, such as a cardiac chamber. Input devices according to the present teachings may also be used with other computer-based medical systems, such as simulation systems for training.

### b. Background

Electrophysiology catheters are used for an ever-increasing number of procedures. For example, catheters have been used for diagnostic, therapeutic, mapping and ablative procedures, to name just a few examples. Typically, a catheter is manipulated through the patient's vasculature to an intended site, for example, a site within the patient's heart, and carries one or more electrodes, which may be used for mapping, ablation, diagnosis, or other treatments.

Traditional techniques of manipulating catheters to, and within, a treatment area typically include a physician manipulating a handle connected to a catheter. The handle generally includes a mechanism directly connected to guide wires for controlling the deflection of a catheter. A second handle is generally provided for controlling deflection of a sheath. Rotating and advancing a catheter or sheath generally requires an electrophysiologist (EP) to physically rotate and advance the associated handle. Deflection of a catheter and sheath generally requires an EP to manipulate a slider switch, a thumb dial, or similar switch which then causes deflection.

Recently, catheter systems have been developed that work in concert with visualization systems, such as the NavX™ system by Saint Jude Medical. However, current methods still generally involve an EP directly and manually controlling a catheter and sheath system, and an associated visualization system typically reactively monitors catheter movement. This leaves the possibility, however small, that an EP could become confused as to which direction to move a deflection switch to obtain a particular direction of catheter deflection. Moreover, these techniques may not provide an EP with a true representation of physical limitations of catheter and sheath movement. Furthermore, direct connection of a handle with a catheter and sheath may lead to hysteresis between the movement of the handle, and the movement of the catheter and sheath.

US 5,318,525 relates to a steerable catheter comprising a catheter shaft having a distal end, a proximal end and a central lumen extending therebetween, wherein a distal portion of the shaft is deflectable, means extending from the proximal end to the distal end for deflecting a distal portion of the shaft about a transverse axis, a core wire extending through the central lumen and attached to the distal end of the shaft, and means for applying torque to a proximal end of the core wire to deflect the distal portion of the shaft about its longitudinal axis without deflecting the proximal portion of the shaft about its longitudinal axis.

WO 2006/120666 A1 relates to a remote control catheterization system comprising user controls, wherein the user controls include an intuitive user interface comprising a handle that can be moved longitudinally, forward and back along a lontitudinal axis, and also can be moved rotationally, in rotation around the longitudinal axis, the intuitive user interface comprising motion sensors that detect longitudinal motion and rotational motion of the handle and convert them to signals, and signal communication circuitry.

US 2005/0016316 A1 relates to a control unit of the type with tensed cables arranged between a base and a movable wrist, including a sheath joined to the base by a double joint, three actuating cables between the sheath and the base and connecting to the base at the same number of connecting points surrounding the sheath, an arm supporting the wrist, sliding into the sheath, and an actuating means between the arm and the sheath.

### BRIEF SUMMARY OF THE INVENTION

The present invention refers to an input device for a robotic catheter system and is defined in appended claim 1. Preferred embodiments are defined in the dependent claims. Systems are provided for receiving user inputs and providing signals representative of the user inputs to a catheter system, which may be a robotic catheter system. An embodiment of the robotic catheter system (also referred to as "the system") may be used, for example, to manipulate the location and/or orientation of sheath and catheter in a treatment area. A treatment area may be a body portion, such as a heart chamber. The system may incorporate a human input device, e.g., a joystick, configured for interaction with a user; an electronic control system that translates motions of the user at the input device into a resulting movement of a catheter tip; and a visualization device that provides a user with real-time or near-real-time positioning information concerning the catheter tip. The input device is similar in structure to an actual catheter and sheath. This provides the user with a more intuitive method of controlling a catheter and sheath in a desired manner.

The input device includes a first handle and a second handle. The first handle may be configured to control a sheath and the second handle may be configured to control a catheter. The first handle and the second handle each include a shaft portion and may each include a contoured distal end. The contoured distal end may be configured to form a grip portion. Grip portions may be disposed at or about the distal end of the respective shaft. The shaft of the first handle is hollow to define a lumen or passageway therethrough. The shaft of the second handle extends through the lumen or passageway defined through the first handle.

Each of the first shaft and the second shaft is configured to be independently moveable in a first plane, such as an x-y deflection plane. Further, each of the first grip portion and the second grip portion may be independently translatable along an axis defined through the shaft portion, e.g., in a z-direction.

According to the invention, each of the first handle and the second handle is connected or coupled to respective guide wires. Guide wires may extend through the shaft of the respective handles and may be connected or coupled at or near the contoured distal end of the respective handle. According to the invention, the shafts include wire ducts or enclosed channels defined therein, through which the guide wires may be disposed. The guide wires are configured such that movement of a handle causes a corresponding tension response in one or more respective guide wires.

In an embodiment, each of the first handle and the second handles may be connected or coupled to five guide wires. Two guide wires may control or correspond to deflection in a first direction (e.g., the x-direction), two guide wires may control or correspond to deflection in a second direction (e.g., the y-direction), and one guide wire may control or correspond to translation (e.g., the z-direction). Accordingly, movement of a handle may result in a tension response (e.g., a tension, a force, or a displacement), in connection with one or more associated guide wires.

According to the present invention, the input device further comprises one or more sensors configured to monitor a force acting on the plurality of guide wires, and to generate a signal representative of the force.

In an embodiment, a plurality of guide wires are individually coupled or connected to a plurality of individual sensors (e.g., each guide wire is connected or coupled to an associated sensor). A sensor may, for instance, be configured to transmit a signal in response to the movement of an associated handle. For example, and without limitation, a sensor may include a potentiometer which may transmit a signal indicative of the displacement of a guide wire. In another embodiment, one or more sensors may be configured to measure a tension response in, or associated with, a guide wire. In a further embodiment, a sensor may comprise, or be coupled to, a motor/encoder configured to respond to movement of an associated handle.

In an embodiment, the first handle and/or the second handle may include a centering function, whereby the handle is returned to an initial or a home position after displacement by a user.

In an embodiment, the shaft of the first handle and/or the second handle may include a plurality of segments having variable stiffness. For example, the first handle may comprise sections having variable stiffness. The relative stiffness of segments may correspond to the stiffness of sections of an associated sheath.

In an embodiment, the input device may include a controller and associated electronics configured to provide an output signal to a computer system indicating the movement of the input device.

### BRIEF DESCRIPTION OF THE DRAWINGS

Figure 1 is an isometric view of a robotic catheter system according to an embodiment.
Figure 2 is an isometric view of an input device according to an embodiment.
Figures 3A-3C are several views of a handle for an input device according to an embodiment.
Figures 4A-4C are several views of a controller for an input device according to an embodiment.
Figure 5 is a graph generally illustrating a relationship between sensors and position in an embodiment.
Figure 6 illustrates an exemplary input system 100 according to an embodiment.

### DETAILED DESCRIPTION OF THE INVENTION

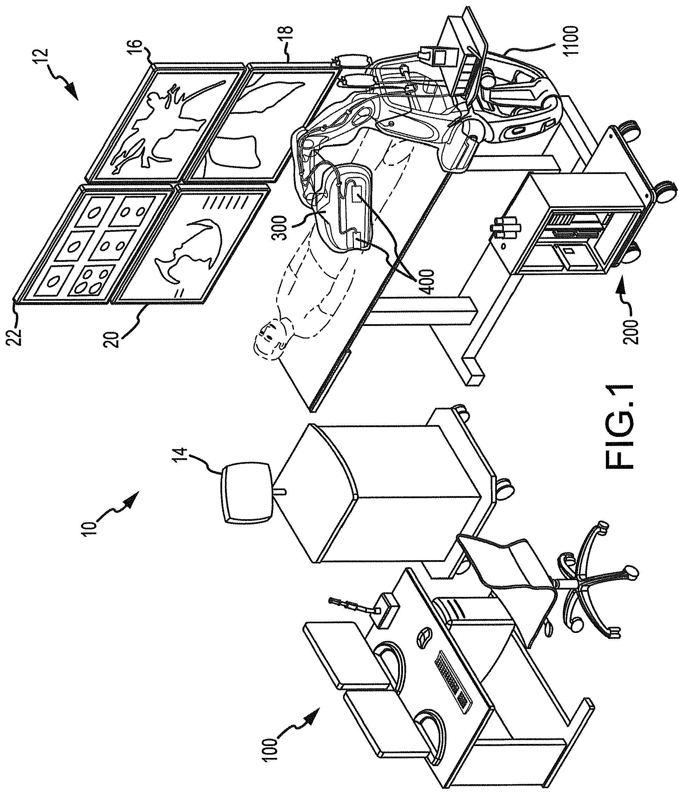

Referring now to the drawings wherein like reference numerals are used to identify like components in the various views, an embodiment of a robotic catheter system 10 (described in detail in co-pending application titled "Robotic Catheter System," published as EP2187806, also referred to as "the system," is illustrated. The system 10 may be used, for example, to manipulate the location and orientation of catheters and sheaths in a treatment area, such as within a heart chamber or another body cavity. As generally illustrated in Figure 1, system 10 may include an input control system 100. Input control system 100 may include an input device, such as a joystick, and related controls (further described below), that a user such as an electrophysiologist (EP) may interact with. Input control system 100 may be coupled to an electronic control system 200 that translates motions of the user at the input device into a resulting movement of a catheter tip. A visualization system 12 may provide a user with real-time or near-real-time positioning information concerning the catheter tip. The system 10 may further include a closed-loop feedback system 14, for example, an EnSite NavX™ system, a magnetic positioning system, and/or optical force transducers. The system 10 may additionally include a robotic catheter manipulator assembly 300 for operating a robotic catheter device cartridge 400, and manipulator support structure 1100. The system 10 provides the user with a similar type of control provided by a conventional manual system, but allows for repeatable, precise, and dynamic movements. In an embodiment, certain elements described above with respect to system 10 may be omitted, or may be combined. For example, while electronic control system 200 is illustrated as a stand-alone unit, it is understood that it may be incorporated into another device, such as manipulator support structure 1100.

Input control system 100 may permit a user to independently control the movement and advancement of a catheter and a sheath. Generally, several types of input devices may be employed. The subject input devices of this teaching include model catheter controls, which may include a first handle and a second handle resembling an oversized sheath and catheter. In an embodiment, by way of example and without limitation, the handles may be self-centering, so that any movement from a center, or home, position causes an incremental movement of the actual catheter tip. In a further embodiment, the input device may work in absolute terms. Haptic feedback may also be employed in connection with the device or system to provide a user with a physical indication associated with contact (e.g., an indication when contact has been made). By way of example, and without limitation, haptic feedback may include heating or cooling a handle of the input device to provide a user with an indication as to electrode temperature, vibrating a handle to indicate, e.g., contact with tissue, and providing resistance to movement of the input device.

Many additional features may be included with the system 10 to, for example, improve the accuracy and/or effectiveness of the system. Such features may include providing feedback using a visualization system 12, or employing a corresponding magnetic positioning system, (e.g., for creating cardiac chamber geometries or models), displaying activation timing and voltage data to identify arrhythmias, and guiding precise catheter movement, and/or optical force transducers. Additional features may include active tensioning of "passive" steering wires to reduce the system response time; cumulative ablation while an electrode tip is following a front-to-back ironing motion; and/or reactive/resistive impedance monitoring.

System 10 may include visualization system 12 which may provide a user with real-time or near-real-time positioning information concerning the catheter tip. In an exemplary embodiment, system 12 may include a monitor 16 for displaying cardiac chamber geometries or models, displaying activation timing and voltage data to identify arrhythmias, and for facilitating guidance of catheter movement. A fluoroscopy monitor 18 may be provided for displaying a real-time x-ray image for assisting a physician with catheter movement. Additional exemplary displays may include an Intracardiac Echo ("ICE") and EP Pruka displays, 20, 22, respectively.

Referring to Figure 1, system 14 will be described briefly.

System 14 (described in detail in U.S. Patent No. 7,263,397, titled "Method and Apparatus for Catheter Navigation and Location and Mapping in the Heart,") may be provided for creating realistic cardiac chamber geometries or models, displaying activation timing and voltage data to identify arrhythmias, and guiding precise catheter movement. System 14 may collect electrical data from catheters, may use this information to track or navigate catheter movement, and to construct three-dimensional (3-D) models of the chamber.

As generally shown in Figure 1, robotic catheter system 10 may include one or more robotic catheter manipulator assemblies 300, for manipulating, for example, catheter and sheath cartridges. Manipulator assembly 300 may include interconnected/interlocking manipulation bases for catheter and sheath cartridges. Each interlocking base may be capable of travel in the longitudinal direction of the catheter/sheath (D₁, D₂ respectively). In an embodiment, D₁ and D₂ may each represent a translation of up to 8 linear inches (20 cm) or more. Each interlocking base may be translated by a high precision drive mechanisms. Such drive mechanism may include, for example and without limitation, a motor driven lead screw or ball screw.

Robotic catheter manipulator assembly 300 may be usable with a robotic catheter rotatable device cartridge. Manipulator base may be replaced with a robotic catheter rotatable drive head and a robotic catheter rotatable drive mechanism.

As briefly discussed above, robotic catheter system 10 may include one or more cartridges 400, with manipulator 300 including at least two cartridges, each of which may be configured to control the distal movement of either the catheter or the sheath. With respect to a catheter cartridge, catheter may be substantially connected or affixed to the cartridge, so that advancement of the cartridge correspondingly advances the catheter, and retraction of the cartridge retracts the catheter. Each cartridge may, for example, include slider blocks rigidly and independently coupled to one of a plurality of catheter steering wires in a manner to permit independent tensioning of each steering wire. The cartridge may be provided as a disposable item that is capable of being easily positioned (e.g., snapped) into place in an overall assembly. In an embodiment, the cartridge may include an electrical "handshake" device or component to allow the system 10 to properly identify the cartridge (e.g., by type and/or proper placement/positioning). A sheath cartridge may be designed in a similar manner as the catheter cartridge, but may be configured to provide for the passage of catheter. The assembly may include a plurality (e.g., as many as ten or more) of independent driving mechanisms (e.g. motor driven ball screws).

Robotic catheter system 10 may be useful for a variety of procedures and in connection with a variety of tools and/or catheters. Such tools and/or catheters may include, without limitation, spiral catheters, ablation catheters, mapping catheters, balloon catheters, transseptal catheters, needle/dilator tools, cutting tools, cauterizing tools, and/or gripping tools. The system 10 may additionally include a means of identifying the nature and/or type of catheter/tool cartridge that is installed for use, and/or position or connection related information. It may also be desirable for the system 10 to automatically access/obtain additional information about the cartridge, such as, without limitation, its creation date, serial number, sterilization date, prior uses, etc.

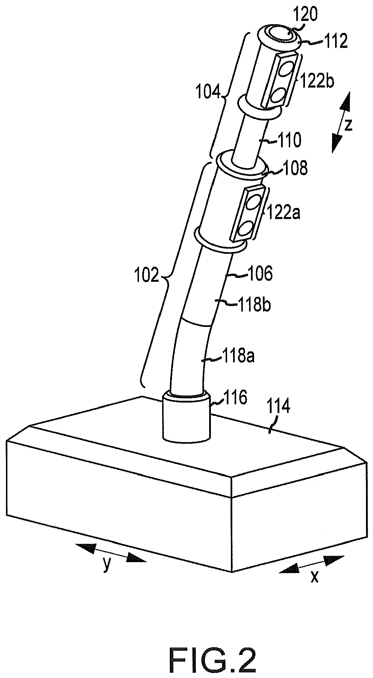

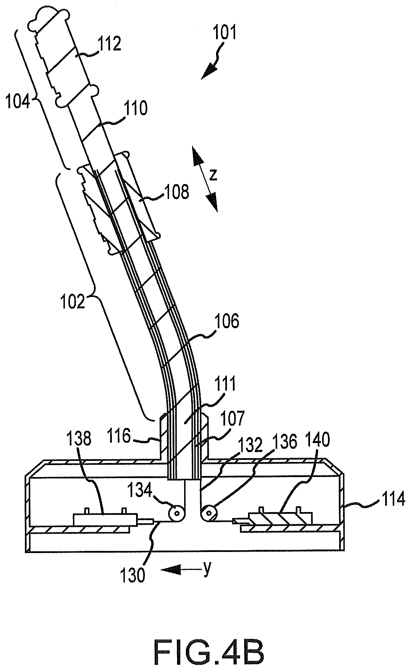

Figure 2 is an isometric view of an embodiment of an input device 101. Input device 101 may be configured to allow a user to independently control a catheter and a sheath. Input device 101 generally includes a first handle 102 and a second handle 104. First handle 102 includes a flexible shaft portion 106 having a proximal end 107 and a first distal end 108 (which may be contoured). Second handle 104 includes a flexible shaft portion 110 having a proximal end 111 and a second distal end 112 (which may be contoured). Proximal end 107 (illustrated in Figure 4B) of first handle 102 and proximal end 111 (illustrated in Figure 4B) of second handle 104 may be securely connected or coupled to a base 114, for example, through a collar 116. First handle 102 and second handle 104 may also include one or more control inputs. For example, the illustrated embodiment of first distal end 108 may include a button 120. Button 120 may be configured to selectively provide one or more functions, such as energizing an ablation electrode of an ablation catheter. First distal end 108 and second distal end 112 may also include one or more input arrays 122a, 122b, which may be configured to control one or more system functions. For example, input array 122a, or 122b, may be configured to serve as a dead man switch.

Base 114 and collar 116 may securely hold the proximal ends 107, 111 of flexible shafts 106, 110. Base 114 may house one or more sensors, for example, as described in further detail below.

Distal end 108 of handle 102, and distal end 112 of handle 104, may be configured to be moveable along a plurality of axes. In an embodiment, distal end 108 of first handle 102 may be configured to be moveable in a first direction, generally illustrated by arrow "x," and in a second direction, generally illustrated by arrow "y." Distal end 108 of first handle 102 may also be configured to be translatable or moveable in a third direction, generally indicated by arrow "z." The direction of translation may be in a direction along shaft 106. Input device 101 may be configured such that movement of distal end 108 of first handle 102 in the x-y plane (the plane defined by the x-arrow and the y-arrow) may result in a corresponding displacement of a sheath within a treatment region. Input device 101 may be further configured such that translation of handle 102 along shaft 106, such as by advancing or retracting distal end 108 along shaft 106, may result in a corresponding advancement or retraction of a sheath.

Similarly, second handle 104 may be configured to be moveable in an x-y plane (such as the plane generally defined by the illustrated x-arrow and y-arrow), and may be configured to be translatable or moveable in a direction along shaft 110. Input device 101 may be further configured such that movement of distal end 112 of second handle 104 within an x-y plane results in a corresponding deflection of a catheter within a treatment area. Translation of handle 104 along shaft 110, such as by advancing or retracting distal end 112 along shaft 110, may result in a corresponding advancement or retraction of a catheter. Moreover, advancement or retraction of distal end 112 of second handle 104 relative to distal end 108 of first handle 102 may result in a corresponding advancement or retraction of a catheter relative to an associated sheath.

As mentioned above, first handle 106 and second handle 110 may include flexible portions or segments. Moreover, first handle 106 and second handle 110 may pivot at a pivot point, such as at proximal end 107 and 111, respectively. Accordingly, movement of a distal end 108 of first handle 102, or distal end 112 of second handle 104, may result in a flexing of an associated shaft 106, 110. It is to be understood that the illustrated x-y plane is provided as a reference only, and that actual movement of distal end 108, 112 of handle 102, 104, may be somewhat non-planar, such as in a partially curved, a partially arcuate, or generally semispherical plane.

In an embodiment, at least one of shaft 106 and shaft 110, may include sections, such as sections 118a, 118b, having varying levels of stiffness, or varying radii of curvature. In an embodiment, the physical properties of a particular section 118a, 118b, such as stiffness or radius of curvature, may correspond to physical properties of an associated section of a sheath.

According to the invention and as will be described in further detail below, input device 101 includes a plurality of guide wires disposed therein. Guide wires are coupled to handles 102, 104 and configured to respond to movement of handles 102, 104. For example, movement of a handle, such as handle 102 or 104, pulls one or more associated guide wires. Pulling a guide wire causes the wire to travel, to stretch, or may otherwise induce a tension response in an associated wire. A tension response in a wire is detected by one or more sensors coupled to the wire. Sensors may include potentiometers, linear actuators, motors, encoders, linear variable displacement transducers, rotary encoders, or other sensors configured to detect a force on, a displacement of, or other tension response in, a guide wire.

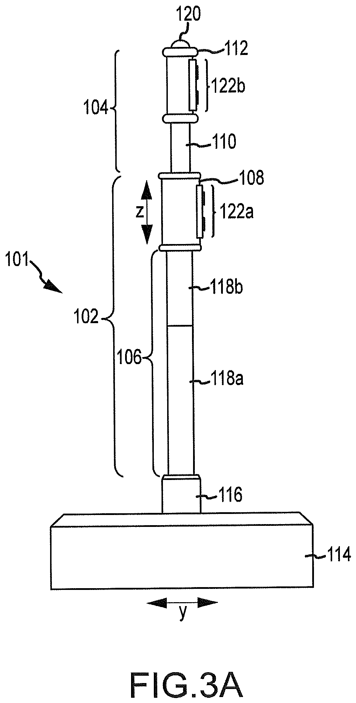

Figures 3A-3C illustrate various side elevation views of an input controller 101, according to an embodiment.

Referring first to Figure 3A, an embodiment of an input device 101 is shown in an exemplary initial, home, or centered position. In an embodiment, sections 118a, 118b of flexible shaft 106, and distal end 108, all of handle 102, as well as flexible shaft 110 and distal end 112 of handle 104 may generally lie in a substantially straight line, along axis z.

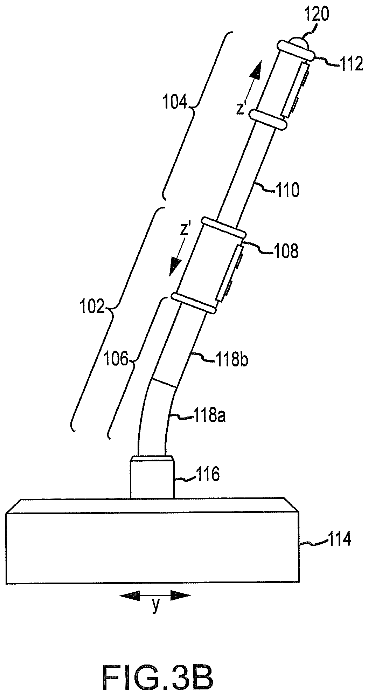

Referring now to Figure 3B, an embodiment of an input device 101 is shown in a position that is off or out of center. Specifically, section 118a of flexible shaft 106 may be flexed along an x-axis. While not directly visible in Figure 3B, it is to be understood that flexible shaft 110 of handle 104 may also be bent proximate section 118a. Furthermore, in the illustrated embodiment, distal end 108 of handle 102, and distal end 112 of handle 104, have been translated along respective shafts 106, 110 generally in opposite directions along an axis z'.

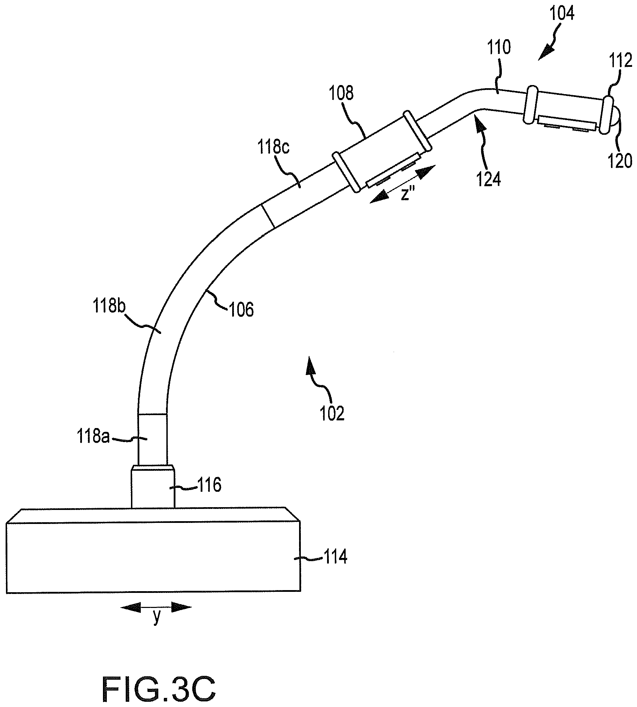

Figure 3C is a further illustration of an embodiment of an input device 101. In the illustrated embodiment, handle 102 is curved along section 118b of flexible shaft 106. As with Figure 3B, it is to be understood that, while not directly visible in Figure 3C, flexible shaft 110 of handle 104 may also be bent proximate section 118b. Flexible shaft 110 may also be curved at a point 124 distal a point where shaft 110 extends past distal end 108 of handle 102.

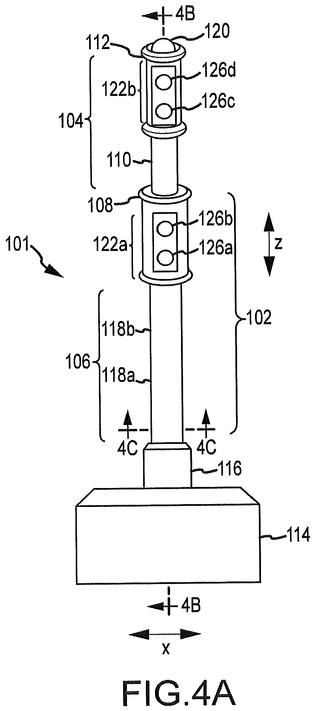

Figure 4A is side view of input device 101 such as generally shown in Figure 2. In the illustrated embodiment, input arrays 122a, 122b, include switches 126a-126d. In an embodiment, switches 126a-126d may include buttons, dials, optical switches, slider switches, or other switches. For example, one or more of switches 126a-126d may be an optical switch or a capacitive switch, which may be configured to serve as a dead man switch.

Figure 4B is a section view of input device 101 along line 4B-4B of Figure 4A. Figure 4B illustrates a first guide wire 130 and a second guide wire 132. First guide wire 130 may be coupled to distal end 108 of handle 102, at or about a first end, and coupled to a sensor 138 at or about a second end. In an embodiment a pulley, such as pulley 134, may facilitate connection of guide wire 130 to distal end 108 and to sensor 138.

Sensor 138 may be configured to output a signal in response to translation of distal end 108 of handle 102. For example, translation of distal end 108 of handle 102 toward distal end 112 of handle 104 may create a tension response in guide wire 130. Sensor 138 may detect a tension response in guide wire 130, and may output a signal indicative of the tension response. For example, sensor 138 may detect a force applied to guide wire 130, and may output a signal indicative of the force. A controller (not pictured) may receive and process the signal to determine the translation of distal end 108.

Similar to guide wire 130, another guide wire 132 may be coupled to flexible shaft 106 and to sensor 140. In an embodiment, a pulley 136 may facilitate coupling of guide wire 132. Sensor 140 may be configured to output a signal in response to deflection of distal end 108 of handle 102 (e.g., in the direction of arrow y). For example, deflection of distal end 108 may create a corresponding tension response in guide wire 132. Sensor 140 may detect a tension in guide wire 130, and may output a signal indicative of the tension. A controller (not pictured) may receive and process the signal to determine deflection.

Sensors 138, 140 may include force sensors, strain sensors, optical encoders, etc. In an embodiment, sensors 138, 140 may include a linear potentiometer. In such an embodiment, translation of distal end 108 may cause guide wire 130 to pull on potentiometer 138. Potentiometer 138 may transmit a signal indicative of the length of travel of guide wire 130. Similarly, in such an embodiment, deflection of distal end 108 of flexible shaft 106 may cause guide wire 132 to pull on, to exert a force on, or to otherwise induce a tension response in potentiometer 140. Potentiometer 140 may then be configured to output a signal indicative of the length of travel of guide wire 132.

While Figure 4B shows only two guide wires 130, 132, and two sensors 138, 140, it is to be understood that input device 101 may include additional guide wires and sensors. For example, in an embodiment, input device 101 may include two guide wires and two sensors to detect deflection of distal end 108 of handle 102 in an x-plane, two guide wires and two sensors to detect deflection of distal end 108 of handle 102 in a y-plane, and a guide wire and sensor to detect translation of distal end 108 of handle 102. Further, input device 101 may include an equal number of guide wires and sensors to detect deflection and translation of distal end 112 of handle 104.

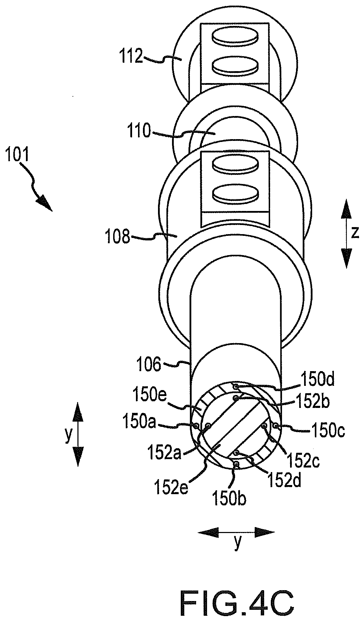

Figure 4C is a partial section view of input device 101 along line 4C-4C of Figure 4A. As generally illustrated, flexible shaft 106 includes a plurality of wire ducts, channels or passages 150a-150e (hereinafter referred to as "wire ducts") through which guide wires may pass. Guide wires associated with wire ducts 150a-150e may be configured to respond to deflection and/or translation of distal end 108 of handle 102. Similarly, flexible shaft 110 includes wire ducts 152a-152e through which guide wires may pass. Guide wires associated with wire ducts 152a-152e may be configured to respond to deflection and/or translation of distal end 112 of handle 104. Certain of the wire ducts may be positioned along an axis corresponding to the axis of deflection to which the associated guide wire is configured to respond. For example, wire ducts 150a and 150c, as well as wire ducts 152a and 152c, may be positioned along an x-axis. Accordingly, the guide wires associated with wire ducts 150a, 150c, 152a, 152c, may be configured to respond to deflection along an x-axis. Wire ducts 150b and 150d, as well as wire ducts 152b and 152d, may be positioned along a y-axis. Accordingly, the guide wires associated with wire ducts 150b, 150d, 152b, 152d, may be configured to respond to deflection along a y-axis. A guide wire associated with wire duct 150e may be configured to respond to translation of distal end 108 of handle 102. A guide wire associated with wire duct 152e may be configured to respond to translation of distal end 112 of handle 104.

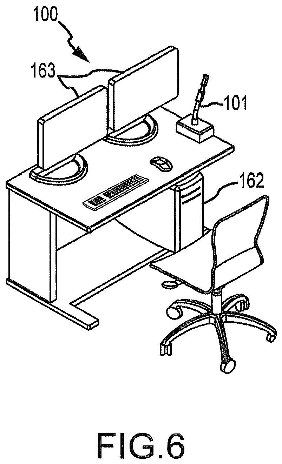

Deflection of distal end 108 of handle 102, and/or deflection of distal end 112 of handle 104, may cause a tension response in a single guide wire, or may cause a deflection of two or more guide wires. Sensors, such as sensors 138, 140, may be configured to transmit signals corresponding to deflection of one or more controllers (e.g., computer 162, discussed below with regard to Figure 6). The one or more controllers may receive the signals, and may thereby determine a location of distal end 108, 112. By comparing a plurality of deflection signals, a controller may be able to determine the location of distal end 108, 112 in the x-y plane.

In an embodiment, base 114 may include a plurality of motors, each coupled to one of a plurality of guide wires. Each of the plurality of motors may be configured to respond to tension in an associated guide wire. Each of the plurality of motors may further be configured to receive one or more signals form a controller. Motors may thereby tension an associated guide wire, which may return an associated handle, such as handle 102, 104, to an initial, a home, or a centered, position.

In a further embodiment, a handle, such as handle 102 and/or handle 104, may include a plurality of guide wires and a rotary controller. For example, a first guide wire and a second guide wire may be configured to respond to deflection along an axis. A rotary controller may be configured to respond to rotation, or deflection, of the distal end 108, 112 of a handle 102, 104.

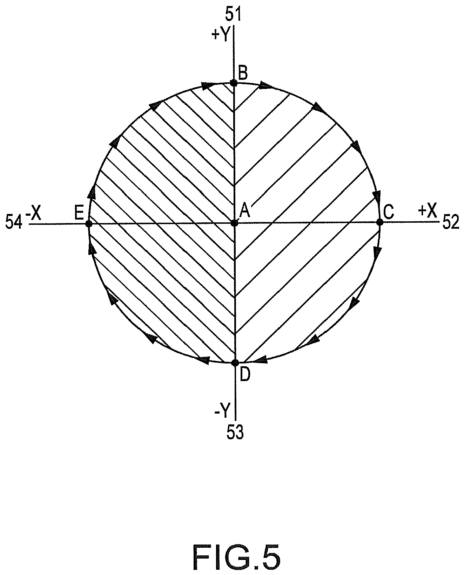

Figure 5 is a graph generally illustrating an exemplary output from each of a plurality of sensors. For example, a first sensor (S1) may be configured to detect deflection in the +y direction. A second sensor (S2) may be configured to detect deflection in the +x direction. A third sensor (S3) may be configured to detect deflection in the -y direction, and a fourth sensor (S4) may be configured to detect deflection in the -x direction. Deflection may be measured from a center, or home location, noted as "A." In the illustrated embodiment, deflection in any given direction is bounded by a respective marker B, C, D, E. While the deflection plane is illustrated as circular, this is merely used as an example, and is not intended to be limiting.

Deflection of distal end 108, 112 along an axis will generally cause a tension response in at least a single guide wire. Deflection in any quadrant of the deflection plane may generally cause a tension response in at least two guide wires, which may be measured by at least two corresponding sensors. For example, deflection into the upper-right quadrant, encompassed by points B and C, will generally cause tension in two guide wires, which may be sensed by sensors S1 and S2. A controller configured to receive signals from sensors S1-S4 may thereby determine the location of distal end 108, 112 of the associated handle 102, 104.

Similarly, a controller, such as computer 162, may be configured to receive signals from a sensor, such as sensor 138, configured to detect translation of distal end 108, 112 along shaft 106, 110. Controller may use the associated signal to further define the location of distal end 108, 112.

An input system 100 is illustrated in figure 6. Input system 100 includes a computing system 162 configured to receive control signals from input device 101, and to display information related to the input control system 100 on one or more displays 163. Displays 163 may be configured to provide visual indications related to patient health, equipment status, catheter position, ablation related information, and/or other information related to catheter procedures. Computing system 162 may be configured to receive signals from input device 101, and to process those signals. For example, computing system 162 may receive signals indicative of a desired motion of a catheter within a patient, may format those signals, and transmit the signals to a manipulator system. The manipulator system may receive the signals and cause a corresponding motion of the catheter. Position, location, and movement of an associated catheter or sheath may be displayed to a user, such as an electrophysiologist, on display 163.

Although embodiments of this invention have been described above with a certain degree of particularity, those skilled in the art could make numerous alterations to the disclosed embodiments without departing from the scope of this invention. For example, while embodiments have been described using potentiometers, it is to be understood that additional embodiment could include other types of sensors and encoders including, without limitation, absolute position encoders, relative position encoders, optical encoders, linear encoders, linear actuators, and linear variable differential transformers. All directional references (e.g., upper, lower, upward, downward, left, right, leftward, rightward, top, bottom, above, below, vertical, horizontal, clockwise, and counterclockwise) are only used for identification purposes to aid the reader's understanding of the present invention, and do not create limitations, particularly as to the position, orientation, or use of the invention. Joinder references (e.g., attached, coupled, connected, and the like) are to be construed broadly and may include intermediate members between a connection of elements and relative movement between elements. As such, joinder references do not necessarily infer that two elements are directly connected and in fixed relation to each other. It is intended that all matter contained in the above description or shown in the accompanying drawings shall be interpreted as illustrative only and not limiting. Changes in detail or structure may be made without departing from the scope of the invention as defined in the appended claims.

## Claims

1. An input device (101) for a robotic catheter system (10), comprising:
a sheath handle (102) comprising a first shaft (106), the first shaft (106) defining a passageway therein; and
a catheter handle (104) comprising a second shaft (110), the second shaft (110) at least partially disposed within the passageway defined by the first shaft (106);
wherein each of the sheath handle (102) and the catheter handle (104) is coupled to a plurality of respective guide wires (130, 132), the guide wires (130, 132) configured such that movement of the handles (102, 104) causes a corresponding tension response in one or more of the plurality of guide wires (130, 132),
further comprising one or more sensors (138, 140) configured to monitor a force acting on the plurality of guide wires (130, 132), and to generate a signal representative of the force,
wherein the sheath handle (102) and the catheter handle (104) each includes one or more wire ducts or channels (150a-d, 152a-d) in which the one or more respective guide wires (130, 132) are disposed.

2. The input device (101) of claim 1, wherein movement of the handles (102, 104) causes or results in movement of one or more of the plurality of guide wires (130, 132).

3. The input device (101) of any one of the preceding claims, wherein the one or more sensors (138, 140) preferably includes at least one of a potentiometer, an optical encoder, a linear variable differential transformer, a rotary encoder, a motor, and a linear actuator.

4. The input device (101) of any one of the preceding claims, wherein the catheter handle (104) further comprises a grip portion coupled to a distal end (112) thereof.

5. The input device (101) of claim 4, wherein the grip portion includes a switch (120, 122a-b, 126a-d), wherein the switch is preferably configured to selectively power an ablation catheter and/or wherein the switch is configured to serve as a dead-man switch.

6. The input device (101) of claim 5, wherein the switch is an optical switch or a capacitive switch.

7. The input device (101) of any one of claims 4 to 6, wherein the grip portion is configured for selective translation along the second shaft (110).

8. The input device (101) of claim 7, further comprising a guide wire coupled to the grip portion, wherein translation of the grip portion results in a force on the guide wire.

9. The input device (101) of any one of the preceding claims, further comprising a controller configured to output signals representative of forces acting on one or more of the plurality of guide wires (130, 132).

10. The input device (101) of claim 1, wherein the first shaft (106) comprises a plurality of segments (118a, 118b), the segments (118a, 118b) having varying levels of stiffness relative to one another, wherein the varying levels of stiffness of the plurality of segments preferably correspond to varying levels of stiffness of an associated sheath.

11. The input device of any one of the preceding claims, wherein the first shaft (106) and the second shaft (110) are at least partially flexible, wherein the flexibility of at least one of the first shaft (106) and the second shaft (110) is preferably representative of the flexibility of an associated sheath and catheter.

12. The input device of any one of the preceding claims, wherein the tension response includes at least one of a tension, a force, or a displacement.

13. A catheter controller, comprising:
an input device (101) according to any one of the preceding claims and a control system (100, 200);
the control system (100, 200) configured to receive control signals in response to movement of the input device (101), and to transmit corresponding motion commands to a catheter;
wherein the input device (101) is configured such that displacement of the input device within a deflection plane results in a corresponding deflection of the distal end of at least one of a catheter and a sheath in a deflection plane.

14. The catheter controller of claim 13, wherein translation of a distal end of the input device (101) results in a corresponding translation of at least one of a catheter and a sheath.

## Patentansprüche

1. Eingabeeinrichtung (101) für ein robotisches Kathetersystem (10), enthaltend:
einen Hüllengriff (102), der einen ersten Schaft (106) enthält, wobei der erste Schaft (106) einen Durchlass in ihm definiert; und
einen Kathetergriff (104), der einen zweiten Schaft (110) enthält, wobei der zweite Schaft (110) zumindest teilweise innerhalb des Durchlasses angeordnet ist, der durch den ersten Schaft (106) definiert wird;
wobei sowohl der Hüllengriff (102) als auch der Kathetergriff (104) mit einer Mehrzahl von jeweiligen Führungsdrähten (130, 132) gekoppelt sind, wobei die Führungsdrähte (130, 132) so konfiguriert sind, dass eine Bewegung der Griffe (102, 104) eine entsprechende Spannungsantwort in einem oder mehreren der Führungsdrähte (130, 132) hervorruft,
weiter enthaltend einen oder mehrere Sensoren (138, 140), die angepasst sind, eine Kraft zu überwachen, die auf die Mehrzahl der Führungsdrähte (130, 132) wirkt und ein Signal zu erzeugen, das repräsentativ für die Kraft ist,
wobei der Hüllengriff (102) und der Kathetergriff (104) jeweils einen oder mehrere Drahtführungen oder Kanäle (150a-d, 152a-d) enthalten, in dem der eine oder die mehreren jeweiligen Führungsdrähte (130, 132) angeordnet sind.

2. Eingabeeinrichtung (101) nach Anspruch 1, wobei eine Bewegung der Griffe (102, 104) eine Bewegung von einem oder mehreren der Mehrzahl der Führungsdrähte (130, 132) bewirkt oder zu dieser führt.

3. Eingabeeinrichtung (101) nach einem der vorhergehenden Ansprüche, wobei der eine Sensor oder die mehreren Sensoren (138, 140) vorzugsweise zumindest einen aus einem Potentiometer, einem optischen Encoder, einem linearvariablen Differentialtransformer, einem Drehencoder, einem Motor und einem Linearaktuator enthalten.

4. Eingabeeinrichtung (101) nach einem der vorhergehenden Ansprüche, wobei der Kathetergriff (104) weiter einen Handgriffbereich enthält, der mit einem distalen Ende (112) davon verbunden ist.

5. Eingabeeinrichtung (101) nach Anspruch 4, wobei der Handgriffbereich einen Schalter (120, 122a-b, 126a-d) enthält, wobei der Schalter vorzugsweise angepasst ist, selektiv einen Ablationskatheter mit Strom zu versorgen, und/oder wobei der Schalter angepasst ist, als ein Toter-Mann-Schalter zu dienen.

6. Eingabeeinrichtung (101) nach Anspruch 5, wobei der Schalter ein optischer Schalter oder ein kapazitiver Schalter ist.

7. Eingabeeinrichtung (101) nach einem der Ansprüche 4 bis 6, wobei der Handgriffbereich zur selektiven Translation entlang des zweiten Schafts (110) angepasst ist.

8. Eingabeeinrichtung (101) nach Anspruch 7, weiter enthaltend einen Führungsdraht, der mit dem Handgriffbereich gekoppelt ist, wobei eine Translation des Handgriffbereichs zu einer Kraft auf den Führungsdraht führt.

9. Eingabeeinrichtung (101) nach einem der vorhergehenden Ansprüche, weiter enthaltend eine Steuerung, die angepasst ist, Signale auszugeben, die repräsentativ für Kräfte sind, die auf einen oder mehrere der Mehrzahl der Führungsdrähte (130, 132) wirken.

10. Eingabeeinrichtung (101) nach Anspruch 1, wobei der erste Schaft (106) eine Mehrzahl von Segmenten (118a, 118b) enthält, wobei die Segmente (118a, 118b) variierende Niveaus von Steifigkeit relativ zueinander haben, wobei die variierenden Niveaus von Steifigkeit der Mehrzahl der Segmente vorzugsweise variierenden Niveaus von Steifigkeit einer zugeordneten Hülle entsprechen.

11. Eingabeeinrichtung nach einem der vorhergehenden Ansprüche, wobei der erste Schaft (106) und der zweite Schaft (110) zumindest teilweise flexibel sind, wobei die Flexibilität von zumindest einem aus dem ersten Schaft (106) und dem zweiten Schaft (110) vorzugsweise repräsentativ für die Flexibilität einer zugehörigen Hülle und eines Katheters ist.

12. Eingabeeinrichtung nach einem der vorhergehenden Ansprüche, wobei die Spannungsantwort zumindest eine aus einer Spannung, einer Kraft oder einer Verschiebung enthält.

13. Kathetersteuerung, enthaltend:
eine Eingabeeinrichtung (101) nach einem der vorhergehenden Ansprüche und ein Steuerungssystem (100, 200);
wobei das Steuerungssystem (100, 200) angepasst ist, Steuersignale in Abhängigkeit von einer Bewegung der Eingabeeinrichtung (101) zu erhalten und entsprechende Bewegungsbefehle an einen Katheter zu übermitteln;
wobei die Eingabeeinrichtung (101) so angepasst ist, dass eine Verschiebung der Eingabeeinrichtung innerhalb einer Verschiebungsebene zu einer entsprechenden Verschiebung des distalen Endes von zumindest einem aus einem Katheter und einer Hülle in einer Verschiebungsebene führt.

14. Kathetersteuerung nach Anspruch 13, wobei eine Translation eines distalen Endes der Eingabeeinrichtung (101) zu einer entsprechenden Translation von zumindest einem aus einem Katheter und einer Hülle führt.

## Revendications

1. Dispositif d'entrée (101) pour un système de cathéter robotisé (10), comprenant :
une poignée de gaine (102) comprenant un premier arbre (106), le premier arbre (106) définissant un passage en son sein ; et
une poignée de cathéter (104) comprenant un second arbre (110), le second arbre (110) étant disposé au moins partiellement à l'intérieur du passage défini par le premier arbre (106) ;
dans lequel chacune de la poignée de gaine (102) et de la poignée de cathéter (104) est couplée à une pluralité de câbles de guidage respectifs (130, 132), les câbles de guidage (130, 132) étant configurés de sorte qu'un mouvement des poignées (102, 104) provoque une réponse en tension correspondante dans un ou plusieurs de la pluralité de câbles de guidage (130, 132),
comprenant en outre un ou plusieurs capteurs (138, 140) configurés pour mesurer une force agissant sur la pluralité de câbles de guidage (130, 132) et pour générer un signal représentatif de la force,
dans lequel la poignée de gaine (102) et la poignée de cathéter (104) incluent chacune un ou plusieurs conduits ou canaux de câble (150a-d, 152a-d) dans lesquels le ou les plusieurs câbles de guidage respectifs (130, 132) sont disposés.

2. Dispositif d'entrée (101) selon la revendication 1, dans lequel un mouvement des poignées (102, 104) provoque ou résulte en un mouvement d'un ou plusieurs de la pluralité de câbles de guidage (130, 132).

3. Dispositif d'entrée (101) selon l'une quelconque des revendications précédentes, dans lequel le ou les plusieurs capteurs (138, 140) incluent de préférence au moins l'un d'un potentiomètre, d'un codeur optique, d'un transformateur différentiel variable linéaire, d'un codeur rotatif, d'un moteur et d'un actionneur linéaire.

4. Dispositif d'entrée (101) selon l'une quelconque des revendications précédentes, dans lequel la poignée de cathéter (104) comprend en outre une portion de saisie couplée à une extrémité distale (112) de celle-ci.

5. Dispositif d'entrée (101) selon la revendication 4, dans lequel la portion de saisie inclut un commutateur (120, 122a-b, 126a-d), dans lequel le commutateur est de préférence configuré pour alimenter de manière sélective un cathéter d'ablation et/ou dans lequel le commutateur est configuré pour servir en tant que commutateur d'homme mort.

6. Dispositif d'entrée (101) selon la revendication 5, dans lequel le commutateur est un commutateur optique ou un commutateur capacitif.

7. Dispositif d'entrée (101) selon l'une quelconque des revendications 4 à 6, dans lequel la portion de saisie est configurée pour une translation sélective le long du second arbre (110).

8. Dispositif d'entrée (101) selon la revendication 7, comprenant en outre un câble de guidage couplé à la portion de saisie, dans lequel la translation de la portion de saisie aboutit à une force sur le câble de guidage.

9. Dispositif d'entrée (101) selon l'une quelconque des revendications précédentes, comprenant en outre une unité de commande configurée pour sortir des signaux représentatifs de forces agissant sur un ou plusieurs de la pluralité de câbles de guidage (130, 132).

10. Dispositif d'entrée (101) selon la revendication 1, dans lequel le premier arbre (106) comprend une pluralité de segments (118a, 118b), les segments (118a, 118b) ayant des niveaux de rigidité variables les uns par rapport aux autres, dans lequel les niveaux de rigidité variables de la pluralité de segments correspondent de préférence à des niveaux de rigidité variables d'une gaine associée.

11. Dispositif d'entrée selon l'une quelconque des revendications précédentes, dans lequel le premier arbre (106) et le second arbre (110) sont au moins partiellement souples, dans lequel la souplesse d'au moins un du premier arbre (106) et du second arbre (110) est de préférence représentative de la souplesse d'une gaine et d'un cathéter associés.

12. Dispositif d'entrée selon l'une quelconque des revendications précédentes, dans lequel la réponse en tension inclut au moins l'un d'une tension, d'une force, ou d'un déplacement.

13. Unité de commande de cathéter, comprenant :
un dispositif d'entrée (101) selon l'une quelconque des revendications précédentes et un système de commande (100, 200) ;
le système de commande (100, 200) étant configuré pour recevoir des signaux de commande en réponse à un mouvement du dispositif d'entrée (101) et pour transmettre des ordres de mouvement correspondant à un cathéter ;
dans lequel le dispositif d'entrée (101) est configuré de sorte qu'un déplacement du dispositif d'entrée à l'intérieur d'un plan de déviation aboutit à une déviation correspondante de l'extrémité distale d'au moins un d'un cathéter et d'une gaine dans un plan de déviation.

14. Unité de commande de cathéter selon la revendication 13, dans laquelle une translation d'une extrémité distale du dispositif d'entrée (101) résulte en une translation correspondante d'au moins un d'un cathéter et d'une gaine.
